# FASCICULE DE BREVET EUROPEEN

(11) **EP 0 430 800 B1**
(45) Date de publication et mention de la délivrance du brevet: **25.01.1995**
(21) Numéro de dépôt: 90403366.9
(22) Date de dépôt: 28.11.1990
(51) Int. Cl.: C07D 277/68, C07D 417/06, A61K 31/425, A61K 31/54

(54) **Nouvelles benzothiazolinones substituées, leur procédé de préparation et les compositions pharmaceutiques qui les contiennent**
Substituierte Benzothiazolinone, ihre Herstellung und sie enthaltende pharmazeutische Zusammenstellungen
Substituted benzothiazolinones, process for their preparation and pharmaceutical compositions containing them

(30) Priorité: 29.11.1989 FR 8915694
(43) Date de publication de la demande: 05.06.1991
(73) Titulaire: ADIR ET COMPAGNIE, 92415 Courbevoie Cédex (FR)
(72) Inventeur: Lesieur, Isabelle, F-59147 Gondecourt (FR); Yous, Said, Laboratoire de Chimie Synthèse, F-59045 Lille Cedex (FR); Devissaguet, Michelle, F-92200 Neuilly sur Seine (FR); Tsouderos, Yannis, F-78170 La Celle Saint-Cloud (FR)

(56) Documents cités:
- EP-A- 0 022 213
- EP-A- 0 040 398
- EP-A- 0 281 309
- CHEMICAL ABSTRACTS 11th COLLECTIVE INDEX, vols. 96-105, 1982-1986, Chemical Substances benzoic acid, chloro-bicyclohexyl, pages 11969CS-11971CS, Columbus, Ohio, US; & CHEMICAL ABSTRACTS, vol.104, no. 11, 17 mars 1986, page 646, résumé no. 88418s; & JP-A-60 166 673 (SUMITOMO CHEMICAL CO., LTD) 29-08-1985
- CHEMICAL ABSTRACTS, vol. 106, Chemical Substance Index, A-Butanedioic, janvier-juin 1987, page 1511CS, Columbus, Ohio, US; & Chemical Abstracts, vol. 106, no. 11, 16 mars 1987, page 228, résumé no. 80382w; & JP-A-61 143 307 (IDEMITSU KOSAN CO., LTD) 01-07-1986

## Description

La présente invention concerne de nouvelles benzothiazolinones substituées, leurs préparations et les compositions pharmaceutiques qui les contiennent.

De nombreux dérivés de la benzothiazolinone ont été décrits en thérapeutique comme possédant des propriétés pharmacologiques très variées.

Le brevet JP 86143307 décrit notamment des alkyl-6 benzothiazolinones en tant que fongicides, la 6-éthyl benzothiazolinone y est mentionnée en tant qu'intermédiaire de synthèse ; le brevet JP 85130574 décrit des amido-6 benzothiazolinones en tant que stimulants cardiaques ; enfin le brevet WO 850 1289 décrit entre autres des acyl-6 benzothiazolinones en tant qu'anti-inflammatoires.

Les brevets FR 2444506 et FR 2491066 décrivent quant à eux des acyl-6 benzoxazolinones utilisables en tant qu'analgésiques.

La demande de brevet EP 0281309 cite les 6-(2-halogéno éthyl) et 6-(4-halogéno butyl) benzothiazolinones en tant qu'intermédiaires de synthèse.

La demanderesse a maintenant découvert des dérivés benzothiazolinoniques doués d'une activité analgésique d'un niveau nettement plus intéressant que celui des dérivés décrits dans le brevet FR 7323280.

En outre les dérivés de l'invention possèdent la caractéristique particulièrement avantageuse d'être totalement dépourvus d'activité anti-inflammatoire et ils se démarquent en cela des dérivés de la demande WO 8501289 : les composés de la présente invention sont en effet doués d'une activité analgésique pure de haut niveau. Or, la plupart des substances analgésiques non morphiniques connues à ce jour possèdent également une activité anti-inflammatoire (exemples : salicylés, pyrazolés ...) et ils interviennent par conséquent sur les processus de l'inflammation. Ceux-ci impliquent de très nombreux médiateurs chimiques (prostaglandines, thromboxane A2...) ; il s'ensuit donc de multiples effets secondaires dont les plus connus sont : attaque de la muqueuse gastrique avec possibilité d'ulcères. Outre les dérangements qu'ils occasionnent, ces effets parallèles interdisent l'usage de ces produits chez de nombreux sujets qui y sont particulièrement sensibles. En étant dépourvus de toute activité anti-inflammatoire, les composés de la présente invention n'interviennent donc pas sur les médiateurs de l'inflammation et sont donc dépourvus des effets secondaires mentionnés précédemment. Cette caractéristique, jointe à leur absence totale de toxicité et à leur haut niveau d'activité rend les composés de la présente invention utilisables en tant qu'analgésique d'une façon beaucoup plus sûre et sans les restrictions d'usage habituellement connues pour la grande majorité de ces produits.

Les produits de l'invention possèdent en outre une activité antiagrégante plaquettaire et peuvent être utilisés dans la prévention des accidents artériels.

Plus spécifiquement, l'invention concerne les dérivés de formule générale (I) : 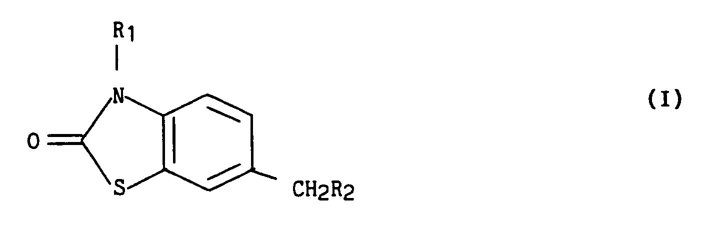
dans laquelle :
R₁ représente un atome d'hydrogène ou un groupement alkyle inférieur,
R₂ représente :
. un groupement alkyle inférieur linéaire ou ramifié éventuellement substitué par un ou plusieurs atomes d'halogène ou par un ou plusieurs groupements alkoxy, hydroxy, phényle, acide carboxylique, ou cyano,
   à la condition que si R₁ représente un atome d'hydrogène alors R₂ ne peut représenter ni un groupement méthyle ni un groupement méthyle substitué par un atome d'halogène,
. un groupement phényle éventuellement substitué par un ou plusieurs :
   - atomes d'halogène, ou groupements cyano,
   - groupements alkyles inférieurs éventuellement substitués par un ou plusieurs atomes d'halogène, ou
   - groupements alkoxy inférieurs, ou acide carboxylique ou hydroxy,
. un groupement hétéroaryle éventuellement substitué par un groupement alkyle inférieur linéaire ou ramifié,
le terme inférieur indiquant que les groupements ainsi qualifiés comptent de 1 à 6 atomes de carbone,
le terme hétéroaryle signifiant groupement insaturé mono ou bicyclique comprenant de 1 à 3 hétéroatomes choisis parmi azote, oxygène ou soufre, chaque cycle comprenant de 5 à 6 sommets,
leurs énantiomères, diastéréoisomères et épimères ainsi que lorsque R₁ représente un atome d'hydrogène ou lorsque R₂ comprend un groupement acide carboxylique leurs sels d'addition à une base pharmaceutiquement acceptable.

De façon plus spécifique, l'invention concerne les dérivés de formule générale (I) dans laquelle :
- R₂ est un groupement phényle éventuellement substitué et R₁ un atome d'hydrogène,
- R₁ est un atome d'hydrogène,
- R₁ est un groupement méthyle et R2 comprend un groupement acide carboxylique,
- R₂ est un groupement alkyle inférieur linéaire ou ramifié substitué par un groupement carboxyle,
- R₂ est un groupement alkyle inférieur linéaire ou ramifié substitué par un atome d'halogène et R₂ est différent d'halogéno méthyle lorsque R₁ est un atome d'hydrogène.
- R₂ est un groupement phényle substitué par un ou plusieurs groupements alkyle inférieur ou alkoxy inférieur ou par un ou plusieurs atomes d'halogène ou par un groupement trifluométhyle et R₁ est un atome d'hydrogène,
ainsi que leurs sels d'addition à une base pharmaceutiquement acceptable.

De façon encore plus spécifique, l'invention concerne la benzyl-6 benzothiazolinone ainsi que leurs sels d'addition à une base pharmaceutiquement acceptable.

Parmi les bases que l'on peut ajouter aux composés de formule (I) pour lesquelles R₁ représente un atome d'hydrogène ou lorsque R₂ comprend un groupement acide carboxylique, on peut citer, à titre d'exemple, les hydroxydes de sodium, potassium, calcium ou des bases organiques comme la diéthylamine, la diéthanolamine, la triéthylamine, le benzylamine, la dicyclohexylamine, l'arginine, ou des carbonates de métaux alcalins ou alcalino terreux.

L'invention s'étend aussi au procédé d'obtention des composés de formule générale (I), caractérisé en ce que l'on utilise comme matière première un dérivé de formule (II) : 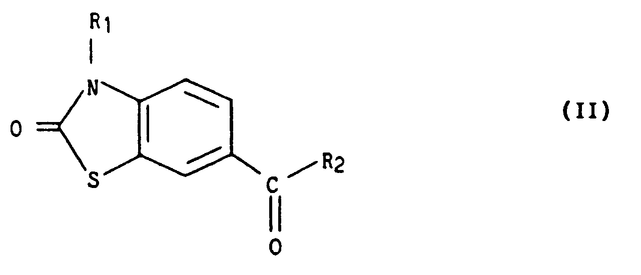
décrit dans la demande de brevet FR 264 6350 dans laquelle R₁ et R₂ ont la même signification que dans la formule (I),
qui, en milieu acide, sont réduits par un trialkylsilane selon la méthode de CT WEST et Coll. (J. Org. Chem. 1973, 38, (15), 2675-2681) pour conduire après purification par une technique choisie parmi lavage, chromatographie et/ou cristallisation au produit de formule (I) : 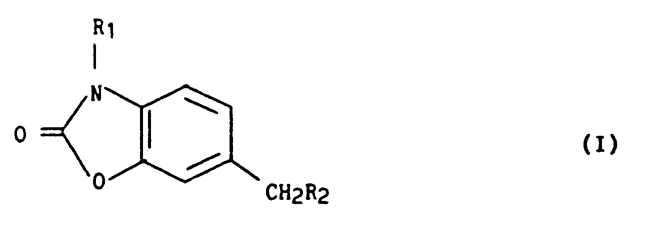
. qui, lorsque R₂ comprend un groupement cyano est appelé dérivé de formule (I/A) et peut être transformé en un dérivé de formule (I) dans lequel R₂ comprend un groupement carboxylique par action sur le dérivé de formule (I/A) précédemment obtenu d'un acide fort,
. qui lorsque R₂ comprend un atome d'halogène peut être traité par un cyanure de métal alcalin pour donner un dérivé de formule (I/A) dans lequel R₂ comprend un groupement cyano, lequel groupement cyano peut être transformé en un groupement carboxyle comme indiqué précédemment pour donner un dérivé de formule (I) dans lequel R₂ comprend un groupement acide carboxylique,
dont on sépare le cas échéant les isomères par une technique classique de séparation et que l'on salifie, si on le désire, lorsque R₁ représente un atome d'hydrogène ou lorsque R₂ comprend un groupement acide carboxylique par une base pharmaceutiquement acceptable.

Les composés de formule (I) possèdent des propriétés pharmacologiques intéressantes.

L'étude pharmacologique des dérivés de l'invention a en effet montré qu'ils étaient peu toxiques, doués d'une activité analgésique de haut niveau et dépourvus d'activité anti-inflammatoire. Ce spectre d'activité rend donc les composés de la présente invention intéressants dans un certain nombre d'indications telles que algies rhumatismales, névralgies, lombosciatiques, névralgies cervico-brachiales, algies traumatiques telles que entorses, fractures, luxations, douleurs post-traumatiques, douleurs post-opératoires, douleurs dentaires, douleurs neurologiques telles que névralgies faciales, douleurs viscérales telles que coliques néphrétiques, dysménorrhées, chirurgie proctologique, douleurs de la sphère O.R.L., pancréatites, algies diverses, céphalées, douleurs des cancéreux. Leur activité antithromboxane permet également leur utilisation dans la prévention des accidents ischémiques artériels périphériques et cérébrovasculaires et dans la prévention et la correction des troubles plaquettaires.

La présente invention a également pour objet les compositions pharmaceutiques contenant les produits de formule (I) ou un de leurs sels d'addition à une base pharmaceutiquement acceptable, seuls ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la voie d'administration, la nature de l'indication thérapeutique ou des traitements éventuellement associés et s'échelonne entre 1 centigramme et 4 grammes par 24 heures.

Les exemples suivants illustrent l' invention.

### EXEMPLE 1 : BENZYL-6 BENZOTHIAZOLINONE

Dans une fiole de 150 cm³, peser 10,2 g (0,04 mole) de benzoyl-6 benzothiazolinone. Ajouter 45,6 g (0,4 mole) d'acide trifluoroacétique par l'intermédiaire d'une ampoule à brome munie d'un robinet en téflon.

Ajouter, sous agitation magnétique, goutte à goutte et en refroidissant au moyen d'un bac d'eau glacée 10,5 g (0,09 mole) de triethylsilane. Adapter une garde à chlorure de calcium et poursuivre l'agitation pendant quinze heures à la température ambiante. Verser, sous agitation, le mélange réactionnel dans 500 cm³ d'eau glacée. Essorer le précipité obtenu, laver à l'eau jusqu'à neutralité des eaux de lavage. Sécher et recristalliser dans le toluène.
Rendement : 95 %
Point de fusion : 140-142°C

| Microanalyse élémentaire : | | | |
|---|---|---|---|
| Calculée → | C : 69,68 | H : 4,59 | N : 5,80 |
| Trouvée → | C : 69,67 | H : 4,46 | N : 5,80 |

### EXEMPLE 2 : METHYL-3 BENZYL-6 BENZOTHIAZOLINONE

Procéder de la même façon que dans l'exemple 1 en remplaçant la benzoyl-6 benzothiazolinone par la méthyl-3 benzoyl-6 benzothiazolinone. Recristalliser dans l'éthanol.
Rendement : 94%
Point de fusion : 114-115 °C

| Microanalyse élémentaire : | | | |
|---|---|---|---|
| Calculée → | C : 70,55 | H : 5,13 | N : 5,48 |
| Trouvée → | C : 69,99 | H : 5,18 | N : 5,47 |

### EXEMPLE 3 : (CHLORO-4 BENZYL)-6 BENZOTHIAZOLINONE

Procéder de la même façon que dans l'exemple 1 en remplaçant la benzoyl-6 benzothiazolinone par la (chloro-4 benzoyl)-6 benzothiazolinone. Recristalliser dans l'acétonitrile.
Rendement : 90%
Point de fusion : 174-176 °C

### EXEMPLE 4 : METHYL-3 (CHLORO-4 BENZYL)- 6 BENZOTHIAZOLINONE

Procéder de la même façon que dans l'exemple 1 en remplaçant la benzoyl-6 benzothiazolinone par la méthyl-3 (chloro-4 benzoyl)-6 benzothiazolinone. Recristalliser dans l'éthanol.
Rendement : 93%
Point de fusion : 105-106 °C

### EXEMPLE 5 : PROPYL-6 BENZOTHIAZOLINONE

Procéder de la même façon que dans l'exemple 1 en remplaçant la benzoyl-6 benzothiazolinone par la propionyl-6 benzothiazolinone.

### EXEMPLE 6 : METHYL-3 PROPYL-6 BENZOTHIAZOLINONE

Procéder de la même façon que dans l'exemple 1 en remplaçant la benzoyl-6 benzothiazolinone par la méthyl-3 propionyl-6 benzothiazolinone.

### EXEMPLE 7 : BUTYL-6 BENZOTHIAZOLINONE

Procéder de la même façon que dans l'exemple 1 en remplaçant la benzoyl-6 benzothiazolinone par la butyryl-6 benzothiazolinone. Recristalliser dans l'acétonitrile.
Rendement : 93 %
Point de fusion : 127-129°C
Caractéristiques spectrales : RMN ¹H Solvant : CDCl₃ 0,95 ppm Triplet, 3H, CH₃

### EXEMPLE 8 : METHYL-3 BUTYL-6 BENZOTHIAZOLINONE

Procéder de la même façon que dans l'exemple 1 en remplaçant la benzoyl-6 benzothiazolinone par la méthyl-3 butyryl-6 benzothiazolinone.

### EXEMPLE 9 : PENTYL-6 BENZOTHIAZOLINONE

Procéder de la même façon que dans l'exemple 1 en remplaçant la benzoyl-6 benzothiazolinone par la valéryl-6 benzothiazolinone. Recristalliser dans le cyclohexanne.
Rendement : 92%
Point de fusion : 80-81 °C

### EXEMPLE 10 : METHYL-3 PENTYL-6 BENZOTHIAZOLINONE

Procéder de la même façon que dans l'exemple 1 en remplaçant la benzoyl-6 benzothiazolinone par la méthyl-3 valéryl-6 benzothiazolinone.

### EXEMPLE 11 : (THIENYL-2 METHYL)-6 BENZOTHIAZOLINONE

Procéder de la même façon que dans l'exemple 1 en remplaçant la benzoyl-6 benzothiazolinone par la (thénoyl-2)-6 benzothiazolinone.

### EXEMPLE 12 : METHYL-3 (THIENYL-2 METHYL)-6 BENZOTHIAZOLINONE

Procéder de la même façon que dans l'exemple 1 en remplaçant la benzoyl-6 benzothiazolinone par la méthyl-3 (thénoyl-2)-6 benzothiazolinone

### EXEMPLE 13 : (HYDROXY-4 BUTYL)-6 BENZOTHIAZOLINONE

Procéder de la même façon que dans l'exemple 1 en remplaçant la benzoyl-6 benzothiazolinone par l'(hydroxy-4 butyryl)-6 benzothiazolinone.

### EXEMPLE 14 : METHYL-3 (HYDROXY-4 BUTYL)-6 BENZOTHIAZOLINONE

Procéder de la même façon que dans l'exemple 13 en remplaçant l'(hydroxy-4 butyryl)-6 benzothiazolinone par la méthyl-3 (hydroxy-4 butyryl)-6 benzothiazolinone.

### EXEMPLE 15 : METHYL-3 ETHYL-6 BENZOTHIAZOLINONE

Procéder de la même façon que dans l'exemple 1 en remplaçant la benzoyl-6 benzothiazolinone par la méthyl-3 acétyl-6 benzothiazolinone.

### EXEMPLE 16 : METHYL-3 (BROMO-2 ETHYL)-6 BENZOTHIAZOLINONE

Procéder de la même façon que dans l'exemple 1 en remplaçant la benzoyl-6 benzothiazolinone par la méthyl-3 (bromoacétyl)-6 benzothiazolinone. Recristalliser dans le cyclohexane.
Rendement : 86%
Point de fusion : 97-98 °C

### EXEMPLE 17 : (CHLORO-3 PROPYL)-6 BENZOTHIAZOLINONE

Procéder de la même façon que dans l'exemple 1 en remplaçant la benzoyl-6 benzothiazolinone par la (chloro-3 propionyl)-6 benzothiazolinone. Recristalliser dans le toluène.
Rendement : 86%
Point de fusion : 41-43 °C

### EXEMPLE 18 : METHYL-3 (CHLORO-3 PROPYL)-6 BENZOTHIAZOLINONE

Procéder de la même façon que dans l'exemple 1 en remplaçant la benzoyl-6 benzothiazolinone par la méthyl-3 (chloro-3 propionyl)-6 benzothiazolinone. Recristalliser dans le cyclohexane.
Rendement : 84 %
Point de fusion : 41-43 °C

### EXEMPLE 19 : (CYANO-3 PROPYL)-6 BENZOTHIAZOLINONE

Dissoudre 0,01 mole de (chloro-3 propyl)-6 benzothiazolinone obtenue dans l'exemple 7 dans 100 cm³ de diméthyl formamide. Ajouter 0,01 mole de CuCN, 0,01 mole de NaCN et 0,02 mole de KCN et agiter une nuit. Evaporer le milieu à sec sous pression réduite. Dissoudre le résidu dans le chloroforme et laver à l'eau à plusieurs reprises. Sécher la phase organique. Evaporer à sec. Recristalliser le résidu.

### EXEMPLE 20 : (CARBOXY-3 PROPYL)-6 BENZOTHIAZOLINONE

Placer 0,01 mole de (cyano-3 propyl)-6 benzothiazolinone dans 50 ml d'acide chlorhydrique dilué au demi. Chauffer à reflux deux heures. Après refroidissement extraire au chloroforme. Sécher sur sulfate de sodium. Evaporer la phase organique. Recristalliser.

### EXEMPLE 21 : METHYL-3 (CYANO-3 PROPYL)-6 BENZOTHIAZOLINONE

Procéder comme dans l'exemple 19 en remplaçant la (chloro-3 propyl)-6 benzothiazolinone par la méthyl-3 (chloro-3 propyl)-6 benzothiazolinone obtenue dans l'exemple 18.

### EXEMPLE 22 : BENZYL-6 BENZOTHIAZOLINONE, SEL DE DIETHANOLANINE

Dans une fiole de 250 cm³, dissoudre 0,04 mole de benzyl-6 benzothiazolinone dans 150 cm³ de dioxanne. Ajouter goutte à goutte 0,04 mole de diethanolanine sous agitation magnétique. Laisser agiter pendant deux heures. Essorer, sécher et recristalliser.

### EXEMPLE 23 : (PHENYL-2 ETHYL)-6 BENZOTHIAZOLINONE

Procéder comme dans l'exemple 1 en remplaçant la benzoyl-6 benzothiazolinone par la phénylacétyl-6 benzothiazolinone.

### EXEMPLE 24 : [(METHYL-4 PHENYL)METHYL]-6 BENZOTHIAZOLINONE

Procéder comme dans l'exemple 1 en remplaçant la benzoyl-6 benzothiazolinone par la p.toluyl-6 benzothiazolinone.

### EXEMPLE 25 : [(TRIFLUOROMETHYL-4 PHENYL)METHYL]-6 BENZOTHIAZOLINONE

Procéder comme dans l'exemple 1 en remplaçant la benzoyl-6 benzothiazolinone par la (trifluorométhyl-4 benzoyl)-6 benzothiazolinone.

### EXEMPLE 26 : P. ANISYL-6 BENZOTHIAZOLINONE

Procéder comme dans l'exemple 1 en remplaçant la benzoyl-6 benzothiazolinone par la p.anisoyl-6 benzothiazolinone.
Point de fusion : 181 - 182°C

### EXEMPLE 27 : (FURYL-2 METHYL)-6 BENZOTHIAZOLINONE

Procéder de la même façon que dans l'exemple 1 en remplaçant la benzoyl-6 benzothiazolinone pa la (furoyl-2)-6 benzothiazolinone.

### EXEMPLE 28 : [(DICHLORO-3,5 PHENYL)METHYL]-6 BENZOTHIAZOLINONE

Procéder de la même façon que dans l'exemple 1 en remplaçant la benzoyl-6 benzothiazolinone par la (dichloro-3,5 benzoyl)-6 benzothiazolinone.

### EXEMPLE 29 : [(TRIMETHOXY-3,4,5 PHENYL)METHYL]-6 BENZOTHIAZOLINONE

Procéder de la même façon que dans l'exemple 1 en remplaçant la benzoyl-6 benzothiazolinone par la (triméthoxy-3,4,5 benzoyl)-6 benzothiazolinone.

### EXEMPLE 30 : (BROMO-4 BUTYL)-6 BENZOTHIAZOLINONE

Procéder de la même façon que dans l'exemple 1 en remplaçant la benzoyl-6 benzothiazolinone par la (bromo-4 butyryl)-6 benzothiazolinone.

### EXEMPLE 31 : (CHLORO-4 BUTYL)-6 BENZOTHIAZOLINONE

Procéder de la même façon que dans l'exemple 1 en remplaçant la benzoyl-6 benzothiazolinone par la (chloro-4 butyryl)-6 benzothiazolinone.

### EXEMPLE 32 : (CHLORO-4 BUTYL)-6 METHYL-3 BENZOTHIAZOLINONE

Procéder de la même façon que dans l'exemple 1 en remplaçant la benzoyl-6 benzothiazolinone par la (chloro-4 butyryl)-6 méthyl-3 benzothiazolinone.

### ETUDE PHARMACOLOGIQUE DES DERIVES DE L'INVENTION

### EXEMPLE 33 : ETUDE DE LA TOXICITE AIGUE

La toxicité aiguë a été appréciée après administration orale à des lots de 5 souris (20 ± 2 grammes) de doses croissantes (0,1 - 0,25 - 0,50 -0,75 - 1 g/kg). Les animaux ont été observés à intervalles réguliers au cours de la première journée et quotidiennement pendant les 2 semaines suivant le traitement.

Il apparaît que les composés de l'invention sont totalement atoxiques. Aucun décès n'est observé après administration d'une dose 1 g.kg⁻¹. On ne constate pas de troubles après administration de cette dose.

### EXEMPLE 34 : ETUDE DE L'ACTIVITE ANALGESIQUE

L'activité sur la douleur a été recherchée chez la souris (20-25 g) selon un protocole dérivé de la technique décrite par SIEGMUND (SIEGMUND E.A., R.A., CADMUS & GOLU, J. Pharm. Exp. Ther. 119, 1874, 1954). Les souris, réparties par randomisation en lots de 12 animaux, ont reçu le traitement par voie orale (excipient pour les témoins) 1 heure avant l'injection intra-péritonéale d'une solution hydroalcoolique de phényl-p-benzoquinone (Sigma) à 0,02%. Les étirements sont dénombrés entre la 5ème et 10ème minute après l'injection.

Le pourcentage d'activité obtenu a été évalué pour chaque dose (% de diminution du nombre d'étirements chez les traités par rapport aux témoins). Une ED₅₀, dose entraînant une activité de 50 %, a été déterminée pour chaque produit.

Il est apparu que certains composés de l'invention possèdent une activité analgésique très intéressante. Ainsi, l'ED₅₀ du composé de l'exemple 1 est voisine de 2 mg.kg⁻¹.

A titre de comparaison l'administration d'une dose de 100 mg.kg⁻¹ des dérivés du brevet Fr 73.23280 provoquait un pourcentage d'analgésie - dans un test comparable - de l'ordre de 25 à 60 % et le composé du brevet Fr 80.20861 dont l'activité analgésique est la plus intéressante avait dans ce même test de Siegmund une ED50 de 9 mg.kg⁻¹ soit environ 4,5 fois supérieure à celle du produit le plus intéressant de la présente invention.

### EXEMPLE 35 : ETUDE DE L'ACTIVITE ANTI-INFLAMMATOIRE

Le potentiel anti-inflammatoire des composés a été recherché sur un modèle d'inflammation aiguë provoquée par injection sous-cutanée d'une solution de carragénine au niveau de la patte postérieure de rat, selon une technique inspirée de la méthode de WINTERCH ; E.A. RISLEY, G.N. NUSS - (Proc. Soc. Exp. Med. 111, 554, 1962). Les rats (100-120 g), randomisés en lots de 8, ont été traités (y compris les témoins qui reçoivent l'excipient) 1 heure avant l'injection locale d'une suspension à 0,5% de carragénine (type IV, Sigma ; 0,1 ml de rat). L'oedème est déterminé 3 heures après l'injection, par mesure pléthysmométrique (pléthysmomètre à eau UGO BASILE) du volume de chacune des pattes postérieures (oedème = volume de la patte enflammée diminué du volume de la patte non enflammmée).

Il apparait que les produits de l'invention n'ont aucune activité sur ce test. En comparaison, les produits du brevet Fr 73.23280 possèdent une activité anti-inflammatoire.

### EXEMPLE 36 : ETUDE DE L'ACTIVITE ANTIAGREGANTE PLAQUETTAIRE

Un plasma riche en plaquettes est préparé à partir de sang humain citraté, provenant de donneurs n'ayant pris aucun médicament pendant les dix jours précédant le prélèvement.
L'agrégation des plaquettes dans ce milieu plasmatique est étudiée par turbidimétrie en employant à des concentrations appropriées l'ADP, l'adrénaline, le collagène, l'acide arachidonique et un agoniste des récepteurs au thromboxane A2, le produit référencé U46119.
Les produits de l'invention sont ajoutés au plasma trois minutes avant l'agoniste.
Les produits de l'invention manifestent une activité antagoniste de l'agrégation plaquettaire.

### EXEMPLE 37 : COMPOSITION PHARMACEUTIQUE : COMPRIMES

| Comprimés dosés à 20 mg de benzyl-6 benzothiazolinone, Formule de préparation pour 1000 comprimés. | |
|---|---|
| Benzyl-6 benzothiazolinone | 20 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

## Revendications (Revendications pour l'(les) Etat(s) contractant(s) suivant(s): AT, BE, CH, DE, DK, FR, GB, IT, LI, LU, NL, SE)

1. Composés de formule générale (I) : 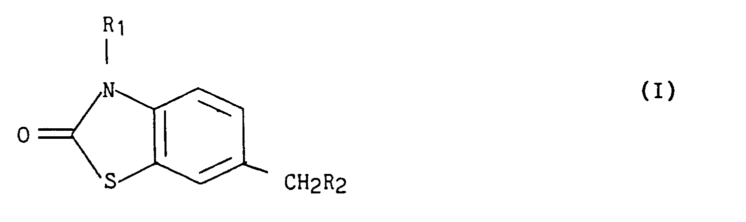 dans laquelle :
R₁ représente un atome d'hydrogène ou un groupement alkyle inférieur,
R₂ représente :
. un groupement alkyle inférieur linéaire ou ramifié éventuellement substitué par un ou plusieurs atomes d'halogène ou par un ou plusieurs groupements alkoxy, hydroxy, phényle, acide carboxylique, ou cyano,
à la condition que si R1 représente un atome d'hydrogène, alors R2 ne peut représenter ni un groupement méthyle, ni un groupement méthyle substitué par un atome d'halogène,
. un groupement phényle éventuellement substitué par un ou plusieurs :
- atomes d'halogène, ou groupements cyano,
- groupements alkyles inférieurs éventuellement substitués par un ou plusieurs atomes d'halogène, ou
- groupements alkoxy inférieurs, ou acide carboxylique ou hydroxy,
. un groupement hétéroaryle éventuellement substitué par un groupement alkyle inférieur linéaire ou ramifié,
le terme inférieur indiquant que les groupements ainsi qualifiés comptent de 1 à 6 atomes de carbone,
le terme hétéroaryle signifiant groupement insaturé mono ou bicyclique comprenant de 1 à 3 hétéroatomes choisis parmi azote, oxygène ou soufre, chaque cycle comprenant de 5 à 6 sommets,
leurs énantiomères, diastéréoisomères et épimères ainsi que, lorsque R₁ représente un atome d'hydrogène ou R₂ comprend un groupement acide carboxylique leurs sels d'addition à une base pharmaceutiquement acceptable.

2. Composés selon la revendication 1, dans laquelle R₂ est un groupement phényle éventuellement substitué ainsi que lorsque R₁ représente un atome d'hydrogène leurs sels d'addition à une base pharmaceutiquement acceptable.

3. Composés selon la revendication 1, dans laquelle R₁ représente un atome d'hydrogène leurs isomères ainsi que leurs sels d'addition à une base pharmaceutiquement acceptable.

4. Composés selon la revendication 1, dans laquelle R₁ représente un groupement méthyle ainsi que leurs isomères et lorsque R₂ comprend un groupement acide carboxylique leurs sels d'addition à une base pharmaceutiquement acceptable.

5. Composés selon la revendication 1 qui est la benzyl-6 benzothiazolinone ainsi que les sels d'addition à une base pharmaceutiquement acceptable.

6. Composés selon la revendication 1 dans lesquels R₂ représente un groupement alkyle inférieur linéaire ou ramifié substitué par un groupement carboxyle ainsi que leurs isomères et leurs sels d'addition à une base pharmaceutiquement acceptable.

7. Composés selon la revendication 1 dans laquelle R₂ représente un groupement alkyle inférieur linéaire ou ramifié substitué par un atome d'halogène et différent d'halogéno méthyle lorsque R₁ représente un atome d'hydrogène ainsi que leurs isomères et lorsque R₁ représente un atome d'hydrogène, leurs sels d'addition à une base pharmaceutiquement acceptable.

8. Composés selon la revendication 1 dans laquelle R₂ représente un groupement phényle substitué par un ou plusieurs groupements alkyle inférieur ou alkoxy inférieur ou par un ou plusieurs atomes d'halogène ou par un groupement trifluorométhyle ainsi que lorsque R₁ représente un atome d'hydrogène, leurs sels d'addition à une base pharmaceutiquement acceptable.

9. Procédé de préparation des composés de formule générale (I), selon la revendication 1, caractérisé en ce que l'on utilise comme matière première un dérivé de formule (II) : 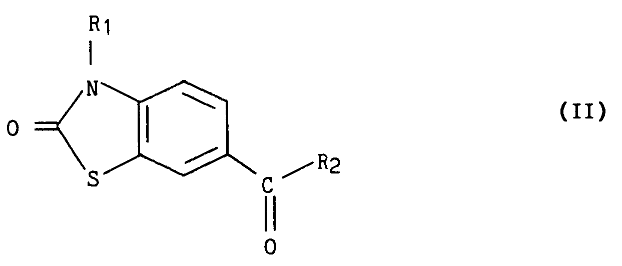 dans laquelle R₁ et R₂ ont la même signification que dans la formule (I),
qui, en milieu acide, sont réduits par un trialkylsilane pour conduire après purification par une technique choisie parmi lavage, chromatographie et/ou cristallisations au produit de formule (I) : 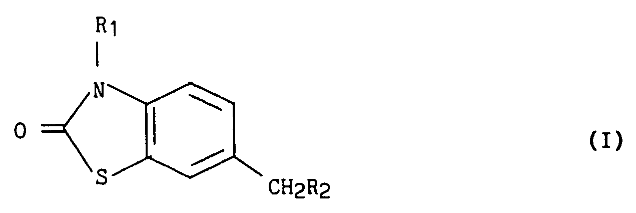 dans laquelle R₁ et R₂ ont la même signification que dans la formule (I),
. qui lorsque R₂ comprend un groupement cyano est appelé dérivé de formule (I/A) et peut être transformé en un dérivé de formule (I) dans lequel R2 comprend un groupement carboxylique par action sur le dérivé de formule (I/A) précédemment obtenu d'un acide fort,
. qui lorsque R₂ comprend un atome d'halogène peut être traité par un cyanure de métal alcalin pour donner un dérivé de formule (I/A) dans lequel R₂ comprend un groupement cyano, lequel groupement cyano peut être transformé en un groupement carboxyle comme indiqué précédemment pour donner un dérivé de formule (I) dans lequel R₂ comprend un groupement acide carboxylique,
dont on sépare le cas échéant les isomères par une technique classique de séparation et que l'on salifie, si on le désire, lorsque R₁ représente un atome d'hydrogène ou lorsque R₂ comprend un groupement acide carboxylique par une base pharmaceutiquement acceptable ou lorsque R₂ comprend un groupement aminé par un acide pharmaceutiquement acceptable.

10. Composition pharmaceutique contenant comme principe actif au moins un composé selon l'une des revendications 1 à 8 en combinaison avec un ou plusieurs excipients ou véhicules inertes, non toxiques, pharmaceutiquement acceptables.

11. Composition pharmaceutique selon la revendication 10 contenant au moins un principe actif selon l'une des revendications 1 à 8 utilisable comme analgésique ainsi que dans la prévention des accidents artériels.

## Revendications (Revendications pour l'(les) Etat(s) contractant(s) suivant(s): ES, GR)

1. Procédé de préparation de composés de formule générale (I) : 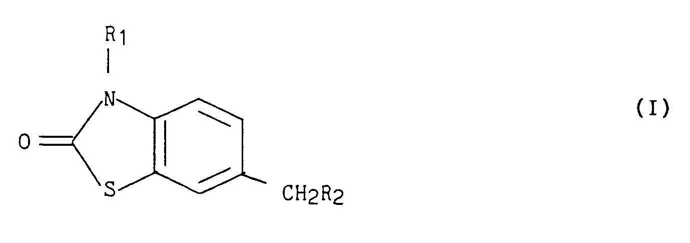 dans laquelle :
R₁ représente un atome d'hydrogène ou un groupement alkyle inférieur,
R₂ représente :
. un groupement alkyle inférieur linéaire ou ramifié éventuellement substitué par un ou plusieurs atomes d'halogène ou par un ou plusieurs groupements alkoxy, hydroxy, phényle, acide carboxylique, ou cyano,
à la condition que si R1 représente un atome d'hydrogène, alors R2 ne peut représenter ni un groupement méthyle, ni un groupement méthyle substitué par un atome d'halogène,
. un groupement phényle éventuellement substitué par un ou plusieurs :
- atomes d'halogène, ou groupements cyano,
- groupements alkyles inférieurs éventuellement substitués par un ou plusieurs atomes d'halogène, ou
- groupements alkoxy inférieurs, ou acide carboxylique ou hydroxy,
. un groupement hétéroaryle éventuellement substitué par un groupement alkyle inférieur linéaire ou ramifié,
le terme inférieur indiquant que les groupements ainsi qualifiés comptent de 1 à 6 atomes de carbone,
le terme hétéroaryle signifiant groupement insaturé mono ou bicyclique comprenant de 1 à 3 hétéroatomes choisis parmi azote, oxygène ou soufre, chaque cycle comprenant de 5 à 6 sommets,
leurs énantiomères, diastéréoisomères et épimères ainsi que, lorsque R₁ représente un atome d'hydrogène ou R₂ comprend un groupement acide carboxylique leurs sels d'addition à une base pharmaceutiquement acceptable,
caractérisé en ce que l'on utilise comme matière première un dérivé de formule (II) : 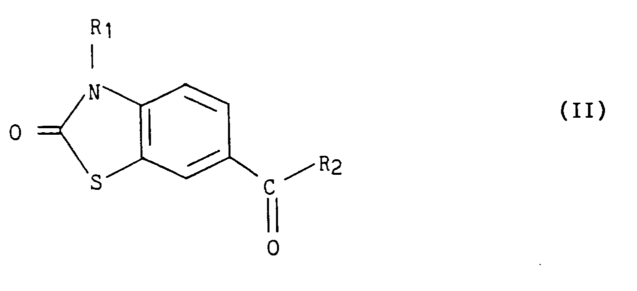 dans laquelle R₁ et R₂ ont la même signification que dans la formule (I),
qui, en milieu acide, sont réduits par un trialkylsilane pour conduire après purification par une technique choisie parmi lavage, chromatographie et/ou cristallisations au produit de formule (I) : 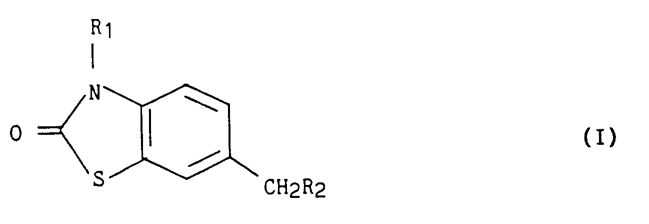 dans laquelle R₁ et R₂ ont la même signification que dans la formule (I),
. qui lorsque R₂ comprend un groupement cyano est appelé dérivé de formule (I/A) et peut être transformé en un dérivé de formule (I) dans lequel R2 comprend un groupement carboxylique par action sur le dérivé de formule (I/A) précédemment obtenu d'un acide fort,
. qui lorsque R₂ comprend un atome d'halogène peut être traité par un cyanure de métal alcalin pour donner un dérivé de formule (I/A) dans lequel R₂ comprend un groupement cyano, lequel groupement cyano peut être transformé en un groupement carboxyle comme indiqué précédemment pour donner un dérivé de formule (I) dans lequel R₂ comprend un groupement acide carboxylique,
dont on sépare le cas échéant les isomères par une technique classique de séparation et que l'on salifie, si on le désire, lorsque R₁ représente un atome d'hydrogène ou lorsque R₂ comprend un groupement acide carboxylique par une base pharmaceutiquement acceptable ou lorsque R₂ comprend un groupement aminé par un acide pharmaceutiquement acceptable.

2. Procédé selon la revendication 1 de préparation de composés de formule (I), dans laquelle R₂ est un groupement phényle éventuellement substitué ainsi que lorsque R₁ représente un atome d'hydrogène leurs sels d'addition à une base pharmaceutiquement acceptable.

3. Procédé selon la revendication 1 de préparation de composés de formule (I), dans laquelle R₁ représente un atome d'hydrogène leurs isomères ainsi que leurs sels d'addition à une base pharmaceutiquement acceptable.

4. Procédé selon la revendication 1 de préparation de composés de formule (I), dans laquelle R₁ représente un groupement méthyle ainsi que leurs isomères et lorsque R₂ comprend un groupement acide carboxylique leurs sels d'addition à une base pharmaceutiquement acceptable.

5. Procédé selon la revendication 1 de préparation du composé de formule (I) qui est la benzyl-6 benzothiazolinone ainsi que les sels d'addition à une base pharmaceutiquement acceptable.

6. Procédé selon la revendication 1 de préparation de composés de formule (I) dans lesquels R₂ représente un groupement alkyle inférieur linéaire ou ramifié substitué par un groupement carboxyle ainsi que leurs isomères et leurs sels d'addition à une base pharmaceutiquement acceptable.

7. Procédé selon la revendication 1 de préparation de composés de formule (I) dans laquelle R₂ représente un groupement alkyle inférieur linéaire ou ramifié substitué par un atome d'halogène et différent d'halogéno méthyle lorsque R₁ représente un atome d'hydrogène ainsi que leurs isomères et lorsque R₁ représente un atome d'hydrogène, leurs sels d'addition à une base pharmaceutiquement acceptable.

8. Procédé selon la revendication 1 de préparation de composé de formule (I) dans laquelle R₂ représente un groupement phényle substitué par un ou plusieurs groupements alkyle inférieur ou alkoxy inférieur, ou par un ou plusieurs atomes d'halogène ou par un groupement trifluorométhyle ainsi que lorsque R₁ représente un atome d'hydrogène, leurs sels d'addition à une base pharmaceutiquement acceptable.

9. Procédé de préparation de compositions pharmaceutiques contenant comme principe actif au moins un composé obtenu selon l'une des revendications 1 à 8 en combinaison avec un ou plusieurs excipients ou véhicules inertes, non toxiques, pharmaceutiquement acceptables.

## Claims (Claims for the following Contracting State(s): AT, BE, CH, DE, DK, FR, GB, IT, LI, LU, NL, SE)

1. Compounds of the general formula (I): 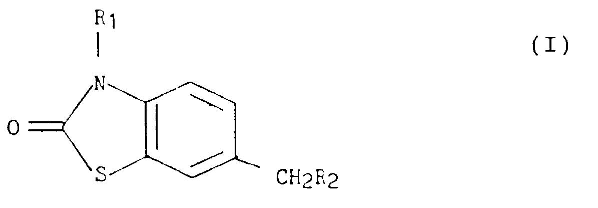 wherein:
R₁ represents a hydrogen atom or a lower alkyl group,
R₂ represents:
. a linear or branched lower alkyl group optionally substituted by one or more halogen atoms or by one or more alkoxy, hydroxy, phenyl, carboxylic acid or cyano groups,
provided that, if R₁ represents a hydrogen atom, R₂ can represent neither a methyl group nor a methyl group substituted by a halogen atom,
. a phenyl group optionally substituted by one or more:
- halogen atoms or cyano groups,
- lower alkyl groups optionally substituted by one or more halogen atoms, or
- lower alkoxy, carboxylic acid or hydroxy groups,
. a heteroaryl group optionally substituted by a linear or branched lower alkyl group,
the term lower indicating that the groups so qualified contain from 1 to 6 carbon atoms, and
the term heteroaryl indicating a mono- or bi-cyclic unsaturated group containing from 1 to 3 hetero atoms selected from nitrogen, oxygen and sulphur, each ring comprising 5 or 6 ring members,
their enantiomers, diastereoisomers and epimers and also, when R₁ represents a hydrogen atom or R₂ contains a carboxylic acid group, their addition salts with a pharmaceutically acceptable base.

2. Compounds according to claim 1 wherein R₂ is an optionally substituted phenyl group and also, when R₁ represents a hydrogen atom, their addition salts with a pharmaceutically acceptable base.

3. Compounds according to claim 1 wherein R₁ represents a hydrogen atom, their isomers and also their addition salts with a pharmaceutically acceptable base.

4. Compounds according to claim 1 wherein R₁ represents a methyl group and also their isomers and, when R₂ contains a carboxylic acid group, their addition salts with a pharmaceutically acceptable base.

5. Compound according to claim 1 that is 6-benzyl-benzothiazolinone and also the addition salts with a pharmaceutically acceptable base.

6. Compounds according to claim 1 wherein R₂ represents a linear or branched lower alkyl group substituted by a carboxy group and also their isomers and their addition salts with a pharmaceutically acceptable base.

7. Compounds according to claim 1 wherein R₂ represents a linear or branched lower alkyl group that is substituted by a halogen atom and is other than halomethyl when R₁ represents a hydrogen atom, and also their isomers and, when R₁ represents a hydrogen atom, their addition salts with a pharmaceutically acceptable base.

8. Compounds according to claim 1 wherein R₂ represents a phenyl group substituted by one or more lower alkyl or lower alkoxy groups or by one or more halogen atoms or by a trifluoromethyl group and also, when R₁ represents a hydrogen atom, their addition salts with a pharmaceutically acceptable base.

9. Process for the preparation of the compounds of the general formula (I) according to claim 1, characterised in that there is used as starting material a compound of formula (II): 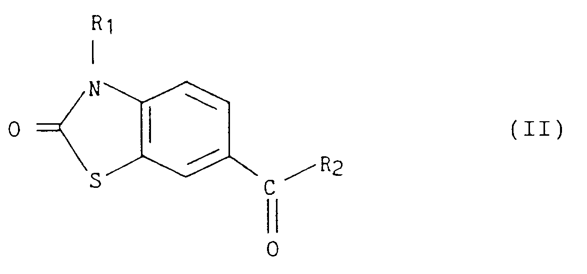 wherein R₁ and R₂ are as defined in formula (I),
which, in an acidic medium, is reduced by a trialkylsilane to yield, after purification by a technique selected from washing, chromatography and/or crystallisations, the product of formula (I): 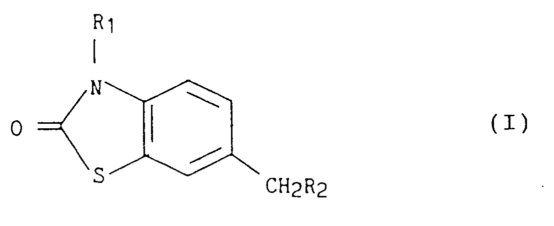 wherein R₁ and R₂ are as defined in formula (I),
. which, when R₂ contains a cyano group, is called the compound of formula (I/A) and can be converted into a compound of formula (I) wherein R₂ contains a carboxylic group by the action of a strong acid on the compound of formula (I/A) previously obtained,
. which, when R₂ contains a halogen atom, can be treated with an alkali metal cyanide to give a compound of formula (I/A) wherein R₂ contains a cyano group, which cyano group can be converted into a carboxy group as indicated above to give a compound of formula (I) wherein R₂ contains a carboxylic acid group,
the isomers of which are optionally separated by a customary separation technique and which is converted into a salt, if desired, when R₁ represents a hydrogen atom or when R₂ contains a carboxylic acid group, using a pharmaceutically acceptable base or, when R₂ contains an amino group, using a pharmaceutically acceptable acid.

10. Pharmaceutical composition containing as active ingredient at least one compound according to one of claims 1 to 8 in combination with one or more pharmaceutically acceptable inert, non-toxic excipients or carriers.

11. Pharmaceutical composition according to claim 10 containing at least one active ingredient according to one of claims 1 to 8 for use as an analgesic and also in the prevention of arterial accidents.

## Claims (Claims for the following Contracting State(s): ES, GR)

1. Process for the preparation of compounds of the general formula (I): 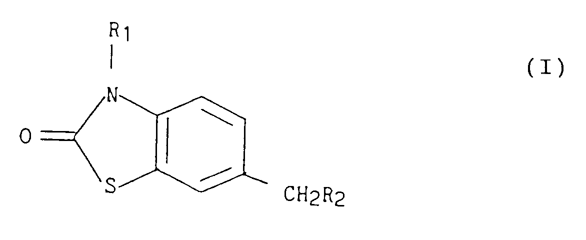 wherein:
R₁ represents a hydrogen atom or a lower alkyl group,
R₂ represents:
. a linear or branched lower alkyl group optionally substituted by one or more halogen atoms or by one or more alkoxy, hydroxy, phenyl, carboxylic acid or cyano groups,
provided that, if R₁ represents a hydrogen atom, R₂ can represent neither a methyl group nor a methyl group substituted by a halogen atom,
. a phenyl group optionally substituted by one or more:
- halogen atoms or cyano groups,
- lower alkyl groups optionally substituted by one or more halogen atoms, or
- lower alkoxy, carboxylic acid or hydroxy groups,
. a heteroaryl group optionally substituted by a linear or branched lower alkyl group,
the term lower indicating that the groups so qualified contain from 1 to 6 carbon atoms, and
the term heteroaryl indicating a mono- or bi-cyclic unsaturated group containing from 1 to 3 hetero atoms selected from nitrogen, oxygen and sulphur, each ring comprising 5 or 6 ring members,
their enantiomers, diastereoisomers and epimers and also, when R₁ represents a hydrogen atom or R₂ contains a carboxylic acid group, their addition salts with a pharmaceutically acceptable base,
characterised in that there is used as starting material a compound of formula (II): 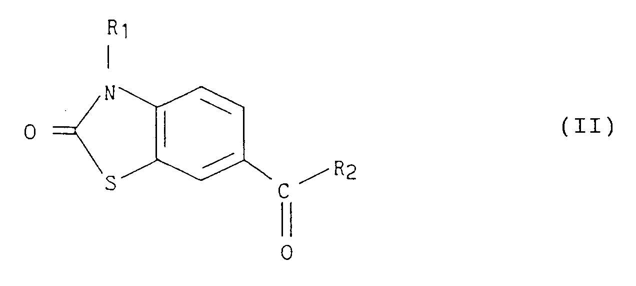 wherein R₁ and R₂ are as defined in formula (I),
which, in an acidic medium, is reduced by a trialkylsilane to yield, after purification by a technique selected from washing, chromatography and/or crystallisations, the product of formula (I): 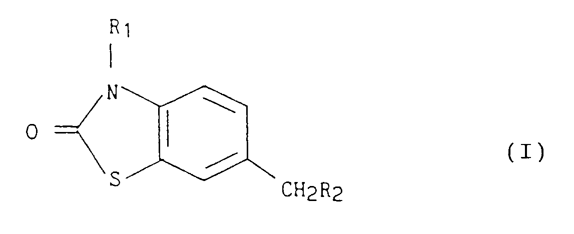 wherein R₁ and R₂ are as defined in formula (I),
. which, when R₂ contains a cyano group, is called the compound of formula (I/A) and can be converted into a compound of formula (I) wherein R₂ contains a carboxylic group by the action of a strong acid on the compound of formula (I/A) previously obtained,
. which, when R₂ contains a halogen atom, can be treated with an alkali metal cyanide to give a compound of formula (I/A) wherein R₂ contains a cyano group, which cyano group can be converted into a carboxy group as indicated above to give a compound of formula (I) wherein R₂ contains a carboxylic acid group,
the isomers of which are optionally separated by a customary separation technique and which is converted into a salt, if desired, when R₁ represents a hydrogen atom or when R₂ contains a carboxylic acid group, using a pharmaceutically acceptable base or, when R₂ contains an amino group, using a pharmaceutically acceptable acid.

2. Process according to claim 1 for the preparation of compounds of formula (I) wherein R₂ is an optionally substituted phenyl group and also, when R₁ represents a hydrogen atom, their addition salts with a pharmaceutically acceptable base.

3. Process according to claim 1 for the preparation of compounds of formula (I) wherein R₁ represents a hydrogen atom, their isomers and also their addition salts with a pharmaceutically acceptable base.

4. Process according to claim 1 for the preparation of compounds of formula (I) wherein R₁ represents a methyl group and also their isomers and, when R₂ contains a carboxylic acid group, their addition salts with a pharmaceutically acceptable base.

5. Process according to claim 1 for the preparation of the compound of formula (I) that is 6-benzylbenzothiazolinone and also the addition salts with a pharmaceutically acceptable base.

6. Process according to claim 1 for the preparation of compounds of formula (I) wherein R₂ represents a linear or branched lower alkyl group substituted by a carboxy group and also their isomers and their addition salts with a pharmaceutically acceptable base.

7. Process according to claim 1 for the preparation of compounds of formula (I) wherein R₂ represents a linear or branched lower alkyl group that is substituted by a halogen atom and is other than halomethyl when R₁ represents a hydrogen atom, and also their isomers and, when R₁ represents a hydrogen atom, their addition salts with a pharmaceutically acceptable base.

8. Process according to claim 1 for the preparation of compounds of formula (I) wherein R₂ represents a phenyl group substituted by one or more lower alkyl or lower alkoxy groups or by one or more halogen atoms or by a trifluoromethyl group and also, when R₁ represents a hydrogen atom, their addition salts with a pharmaceutically acceptable base.

9. Process for the preparation of pharmaceutical compositions containing as active ingredient at least one compound obtained according to one of claims 1 to 8 in combination with one or more pharmaceutically acceptable inert, non-toxic excipients or carriers.

## Patentansprüche (Patentansprüche für folgende(n) Vertragsstaat(en): AT, BE, CH, DE, DK, FR, GB, IT, LI, LU, NL, SE)

1. Verbindungen der allgemeinen Formel (I): 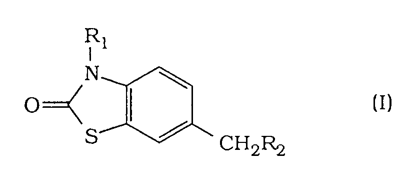 in der
R₁ ein Wasserstoffatom oder eine niedrigmolekulare Alkylgruppe,
R₂
· eine geradkettige oder verzweigte niedrigmolekulare Alkylgruppe, die gegebenenfalls durch eines oder mehrere Halogenatome oder durch eine oder mehrere Alkoxy-, Hydroxyl-, Phenyl-, Carbonsäure- oder Cyano-gruppen substituiert ist,
mit der Maßgabe, daß wenn R₁ ein Wasserstoffatom darstellt, R₂ weder eine Methylgruppe noch eine durch ein Halogenatom substituierte Methylgruppe bedeutet,
· eine Phenylgruppe, die gegebenenfalls durch eines oder mehrere:
- Halogenatome oder Cyanogruppen,
- niedrigmolekulare Alkylgruppen, die gegebenenfalls durch eines oder mehrere Halogenatome substituiert sind, oder
- niedrigmolekulare Alkoxygruppen, Carbonsäuregruppen oder Hydroxylgruppen,
substituiert sind, oder
· eine Heteroarylgruppe, die gegebenenfalls durch eine geradkettige oder verzweigte niedrigmolekulare Alkylgruppe substituiert ist,
wobei der Begriff niedrigmolekular bedeutet, daß die in dieser Weise bezeichneten Gruppen 1 bis 6 Kohlenstoffatome aufweisen,
wobei der Begriff Heteroarylgruppe für eine ungesättigte, mono- oder bicyclische Gruppe mit 1 bis 3 Heteroatomen ausgewählt aus Stickstoff, Sauerstoff oder Schwefel, wobei jeder Ring 5 bis 6 Ringglieder aufweist, steht,
deren Enantiomere, Diastereoisomere und Epimere sowie, wenn R₁ ein Wasserstoffatom oder R₂ eine Carbonsäuregruppe bedeutet, deren Additionssalze mit einer pharmazeutisch annehmbaren Base.

2. Verbindungen nach Anspruch 1, worin R₂ eine gegebenenfalls substituierte Phenylgruppe darstellt, sowie, wenn R₁ ein Wasserstoffatom bedeutet, deren Additionssalze mit einer pharmazeutisch annehmbaren Base.

3. Verbindungen nach Anspruch 1, worin R₁ ein Wasserstoffatom darstellt, deren Isomeren sowie deren Additionssalze mit einer pharmazeutisch annehmbaren Base.

4. Verbindungen nach Anspruch 1, worin R₁ eine Methylgruppe bedeutet, sowie deren Isomeren und, wenn R₂ eine Carbonsäuregruppe darstellt, deren Additionssalze mit einer pharmazeutisch annehmbaren Base.

5. Verbindungen nach Anspruch 1, nämlich 6-Benzyl-benzothiazolinon sowie dessen Additionssalze mit einer pharmazeutisch annehmbaren Base.

6. Verbindungen nach Anspruch 1, worin R₂ eine geradkettige oder verzweigte niedrigmolekulare Alkylgruppe, die durch eine Carbonsäuregruppe substituiert ist, bedeutet sowie deren Isomeren und deren Additionssalze mit einer pharmazeutisch annehmbaren Base.

7. Verbindungen nach Anspruch 1, worin R₂ eine geradkettige oder verzweigte niedrigmolekulare Alkylgruppe, die durch ein Halogenatom substituiert ist, darstellt und von einer Halogenmethylgruppe verschieden ist, wenn R₁ ein Wasserstoffatom bedeutet, sowie deren Isomeren und wenn R₁ ein Wasserstoffatom darstellt, deren Additionssalze mit einer pharmazeutisch annehmbaren Base.

8. Verbindungen nach Anspruch 1, worin R₂ eine Phenylgruppe, die durch eine oder mehrere niedrigmolekulare Alkylgruppen oder niedrigmolekulare Alkoxygruppen oder durch eines oder mehrere Halogenatome oder durch eine Trifluormethylgruppe substituiert ist, darstellt, sowie, wenn R₁ ein Wasserstoffatom bedeutet, deren Additionssalze mit einer pharmazeutisch annehmbaren Base.

9. Verfahren zur Herstellung der Verbindungen der allgemeinen Formel (I) nach Anspruch 1, **dadurch gekennzeichnet**, daß man als Ausgangsmaterial ein Derivat der Formel (II) verwendet: 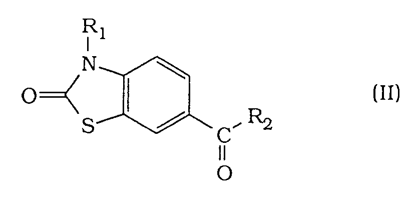 in der R₁ und R₂ die bezüglich der Formel (I) angegebenen Bedeutungen besitzen, welches in saurem Medium mit einem Trialkylsilan reduziert wird, so daß man nach der Reinigung mit Hilfe einer Methode ausgewählt aus Waschen, Chromatographie und/oder Kristallisation, das Produkt der Formel (I) erhält: 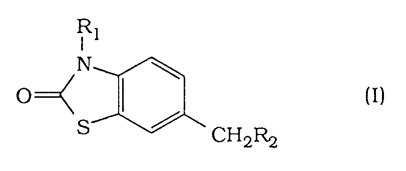 in der R₁ und R₂ die bezüglich der Formel (I) angegebenen Bedeutungen besitzen,
· welches, wenn R₂ eine Cyanogruppe darstellt, als Derivat der Formel (I/A) bezeichnet wird und welches durch Einwirkenlassen einer starken Säure auf das zuvor erhaltene Derivat der Formel (I/A) in ein Derivat der Formel (I) umgewandelt werden kann, in der R₂ eine Carbonsäuregruppe umfaßt,
· welches, wenn R₂ ein Halogenatom darstellt, mit einem Alkalimetallcyanid behandelt werden kann zur Bildung eines Derivats der Formel (I/A), worin R₂ eine Cyanogruppe darstellt, welche Cyanogruppe wie oben angegeben in eine Carboxylgruppe umgewandelt werden kann zur Bildung eines Derivats der Formel (I), in der R₂ eine Carbonsäuregruppe umfaßt,
welches man gegebenenfalls mit Hilfe einer klassischen Trennmethode in die Isomeren auftrennt und, wenn R₁ ein Wasserstoff darstellt oder R₂ eine Carbonsäuregruppe umfaßt, gewünschtenfalls mit einer pharmazeutisch annehmbaren Base oder, wenn R₂ eine Aminogruppe darstellt, mit einer pharmazeutisch annehmbaren Säure in ein Salz überführt.

10. Pharmazeutische Zubereitung enthaltend als Wirkstoff mindestens eine Verbindung nach einem der Ansprüche 1 bis 8 in Kombination mit einem oder mehreren inerten, nichttoxischen, pharmazeutisch annehmbaren Trägermaterialien oder Bindemitteln.

11. Pharmazeutische Zubereitung nach Anspruch 10 enthaltend mindestens einen Wirkstoff nach einem der Ansprüche 1 bis 8 verwendbar als Analgetikum sowie zur Vorbeugung von Arterienvorfällen.

## Patentansprüche (Patentansprüche für folgende(n) Vertragsstaat(en): ES, GR)

1. Verfahren zur Herstellung von Verbindungen der allgemeinen Formel (I): 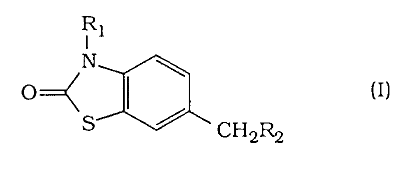 in der
R₁ ein Wasserstoffatom oder eine niedrigmolekulare Alkylgruppe,
R₂:
· eine geradkettige oder verzweigte niedrigmolekulare Alkylgruppe, die gegebenenfalls durch eines oder mehrere Halogenatome oder durch eine oder mehrere Alkoxy-, Hydroxyl-, Phenyl-, Carbonsäure- oder Cyano-gruppen substituiert ist,
mit der Maßgabe, daß wenn R₁ ein Wasserstoffatom darstellt, R₂ weder eine Methylgruppe noch eine durch ein Halogenatom substituierte Methylgruppe bedeutet,
· eine Phenylgruppe, die gegebenenfalls durch eines oder mehrere:
- Halogenatome oder Cyanogruppen,
- niedrigmolekulare Alkylgruppen, die gegebenenfalls durch eines oder mehrere Halogenatome substituiert sind, oder
- niedrigmolekulare Alkoxygruppen, Carbonsäuregruppen oder Hydroxylgruppen,
substituiert sind, oder
· eine Heteroarylgruppe, die gegebenenfalls durch eine geradkettige oder verzweigte niedrigmolekulare Alkylgruppe substituiert ist,
wobei der Begriff niedrigmolekular bedeutet, daß die in dieser Weise bezeichneten Gruppen 1 bis 6 Kohlenstoffatome aufweisen,
wobei der Begriff Heteroarylgruppe für eine ungesättigte, mono- oder bicyclische Gruppe mit 1 bis 3 Heteroatomen ausgewählt aus Stickstoff, Sauerstoff oder Schwefel, wobei jeder Ring 5 bis 6 Ringglieder aufweist, steht,
von deren Enantiomeren, Diastereoisomeren und Epimeren sowie, wenn R₁ ein Wasserstoffatom oder R₂ eine Carbonsäuregruppe bedeutet, deren Additionssalzen mit einer pharmazeutisch annehmbaren Base,
**dadurch gekennzeichnet**, daß man als Ausgangsmaterial ein Derivat der Formel (II) verwendet: 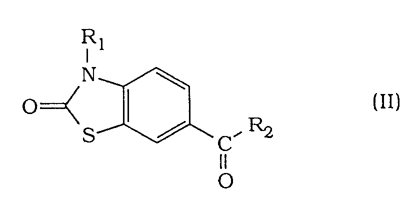 in der R₁ und R₂ die bezüglich der Formel (I) angegebenen Bedeutungen besitzen, welches in saurem Medium mit einem Trialkylsilan reduziert wird, so daß man nach der Reinigung mit Hilfe einer Methode ausgewählt aus Waschen, Chromatographie und/oder Kristallisation, das Produkt der Formel (I) erhält: 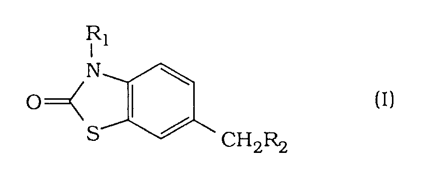 in der R₁ und R₂ die bezüglich der Formel (I) angegebenen Bedeutungen besitzen,
· welches, wenn R₂ eine Cyanogruppe darstellt, als Derivat der Formel (I/A) bezeichnet wird und welches durch Einwirkenlassen einer starken Säure auf das zuvor erhaltene Derivat der Formel (I/A) in ein Derivat der Formel (I) umgewandelt werden kann, in der R₂ eine Carbonsäuregruppe umfaßt,
· welches, wenn R₂ ein Halogenatom darstellt, mit einem Alkalimetallcyanid behandelt werden kann zur Bildung eines Derivats der Formel (I/A), worin R₂ eine Cyanogruppe darstellt, welche Cyanogruppe wie oben angegeben in eine Carboxylgruppe umgewandelt werden kann zur Bildung eines Derivats der Formel (I), in der R₂ eine Carbonsäuregruppe umfaßt,
welches man gegebenenfalls mit Hilfe einer klassischen Trennmethode in die Isomeren auftrennt und, wenn R₁ ein Wasserstoff darstellt oder R₂ eine Carbonsäuregruppe umfaßt, gewünschtenfalls mit einer pharmazeutisch annehmbaren Base oder, wenn R₂ eine Aminogruppe darstellt, mit einer pharmazeutisch annehmbaren Säure in ein Salz überführt.

2. Verfahren nach Anspruch 1 zur Herstellung von Verbindungen der Formel (I), in der R₂ eine gegebenenfalls substituierte Phenylgruppe bedeutet, und von deren Additionssalzen mit einer pharmazeutisch annehmbaren Base, wenn R₁ ein Wasserstoffatom darstellt.

3. Verfahren nach Anspruch 1 zur Herstellung von Verbindungen der Formel (I), in der R₁ ein Wasserstoffatom bedeutet, von deren Isomeren sowie von deren Additionssalzen mit einer pharmazeutisch annehmbaren Base.

4. Verfahren nach Anspruch 1 zur Herstellung von Verbindungen der Formel (I), in der R₁ eine Methylgruppe bedeutet, sowie von deren Isomeren und, wenn R₂ eine Carbonsäuregruppe darstellt, von deren Additionssalzen mit einer pharmazeutisch annehmbaren Base.

5. Verfahren nach Anspruch 1 zur Herstellung von Verbindungen der Formel (I), nämlich von 6-Benzyl-benzothiazolinon sowie von dessen Additionssalzen mit einer pharmazeutisch annehmbaren Base.

6. Verfahren nach Anspruch 1 zur Herstellung von Verbindungen der Formel (I), in der R₂ eine geradkettige oder verzweigte niedrigmolekulare Alkylgruppe, die durch eine Carbonsäuregruppe substituiert ist, bedeutet, sowie von deren Isomeren und deren Additionssalzen mit einer pharmazeutisch annehmbaren Base.

7. Verfahren nach Anspruch 1 zur Herstellung von Verbindungen der Formel (I), in der R₂ eine geradkettige oder verzweigte niedrigmolekulare Alkylgruppe bedeutet, die durch ein Halogenatom substituiert ist und von einer Halogenmethylgruppe verschieden ist, wenn R₁ ein Wasserstoffatom bedeutet, sowie von deren Isomeren und, wenn R₁ ein Wasserstoffatom darstellt, von deren Additionssalzen mit einer pharmazeutisch annehmbaren Base.

8. Verfahren nach Anspruch 1 zur Herstellung der Verbindung der Formel (I), in der R₂ eine Phenylgruppe bedeutet, die durch eine oder mehrere niedrigmolekulare Alkylgruppen oder niedrigmolekulare Alkoxygruppen oder durch eines oder mehrere Halogenatome oder durch eine Trifluormethylgruppe substituiert ist, sowie, wenn R₁ ein Wasserstoffatom darstellt, von deren Additionssalzen mit einer pharmazeutisch annehmbaren Base.

9. Verfahren zur Herstellung von pharmazeutischen Zubereitungen, die als Wirkstoff mindestens eine Verbindung, erhalten nach einem der Ansprüche 1 bis 8, in Kombination mit einem oder mehreren inerten, nichttoxischen, pharmazeutisch annehmbaren Trägermaterialien oder Bindemitteln enthalten.
